# EUROPEAN PATENT APPLICATION

(11) **EP 4 026 713 A1**
(43) Date of publication of application: **13.07.2022**
(21) Application number: 22150443.4
(22) Date of filing: 06.01.2022
(51) Int. Cl.: B60H 3/00, A61L 9/12

(54) **FRAGRANCE SYSTEM AND CONTROL METHOD THEREOF**

(30) Priority: 08.01.2021 CN 202110025408
(71) Applicant: Nio Technology (Anhui) Co., Ltd, Hefei City, Anhui 230601 (CN)
(72) Inventor: HAN, Fang, AnTing Town, Jiading Shanghai (CN); GONG, Haining, AnTing Town, Jiading Shanghai (CN); HE, Yukun, AnTing Town, Jiading Shanghai (CN); CHEN, Ming, AnTing Town, Jiading Shanghai (CN); WANG, Xueshi, AnTing Town, Jiading Shanghai (CN)
(74) Representative: Patentwerk B.V.

(57) **Abstract**

A fragrance system comprising a fragrance module including a housing and at least one fragrance chamber disposed in the housing, wherein an NFC antenna (14) is configured above each fragrance chamber, and a fragrance unit (2), which is configured in the fragrance chamber in a removable manner and able to send forth a fragrance corresponding to the fragrance unit (2) into a target space when the fragrance module is enabled, wherein the fragrance unit (2) is provided with an NFC tag that is loaded with identity information of the fragrance unit (2) and the NFC tag is disposed in the fragrance unit (2) in a built-in manner.

## Description

### Technical Field

The invention relates to the field of fragrance technologies, and specifically, to a fragrance system and a control method thereof, a fragrance unit, a vehicle, a computer storage medium, and a control apparatus.

### Background Art

As the automobile industry develops, there are not only higher requirements on technologies related to traveling performance and environment friendliness, but also requirements for satisfying individual demands during driving or riding from the perspective of a driver or passenger (user). For example, one of the individual demands is to introduce a fragrance system into a vehicle cabin, so that a user can enjoy a scented environment.

A process of implementing functions of the fragrance system is roughly as follows: A fragrance bottle with a scent is mounted (for example, plugged) onto a fragrance module of a vehicle through matching. In this way, when the fragrance module is enabled, a corresponding scent can spread over a vehicle cabin of the vehicle. To cater to demands of a user, there are more and more types of scents in fragrance bottles in the market, and to ensure that a fragrance can be accurately recognized and tracked, the following manners, for example, of recognition have appeared in the market:

(1) SIM card recognition: In this recognition solution, a SIM card would be exposed on the surface of a fragrance bottle, which is unfavorable to smoothness of the product. In addition, because there is a common lack of automotive-grade SIM cards at present and that a fragrance bottle usually has to be exposed in a relatively complex vehicle cabin environment for a long time during a service life, a problem that a SIM card is poorly connected or even becomes ineffective may be caused.

(2) Resistance recognition: In this recognition solution, after a fragrance bottle is mounted onto a fragrance module through matching, it needs to be ensured that: an electrically conductive structure on the top can still stay in stable physical contact with the fragrance module even in the case of a complex traffic condition and after the fragrance bottle is plugged and unplugged a plurality of times. This inevitably proposes higher requirements on production precision of a whole fragrance system.

Accordingly, there is a need for a new technical solution to solve the foregoing problems in the art.

### Summary of the Invention

To solve the foregoing problems in the prior art, a first aspect of the invention provides a fragrance system based on near field communication (NFC), and the fragrance system includes: a fragrance module including a housing and at least one fragrance chamber disposed in the housing, where an NFC antenna is configured above each fragrance chamber; and a fragrance unit, which is able to send forth a fragrance corresponding to the fragrance unit into target space when the fragrance module is enabled, where the fragrance unit is configured in the fragrance chamber in a removable manner, and the fragrance unit is provided with an NFC tag that is loaded with identity information of the fragrance unit, where the NFC tag is disposed in the fragrance unit in a built-in manner.

In this way, while the fragrance unit can be recognized based on an NFC technology, smoothness of the fragrance bottle is ensured and durability of a whole vehicle is optimized.

It can be understood that those skilled in the art may select a specific built-in manner based on actual conditions, which may be, for example, as follows: The NFC tag is injection-molded directly on an inner side of the top of the fragrance bottle; or the NFC tag is stuck directly onto an inner side of the top of the fragrance bottle by using gum. Compared with other recognition manners, use of an NFC tag simplifies an assembly procedure. Compared with sticking the NFC tag to the inside of the fragrance bottle, direct injection molding into the fragrance bottle makes the tag more fixed.

For the foregoing fragrance system based on NFC, in a possible implementation, the fragrance unit includes: a fragrance bottle formed with an accommodation cavity; and a fragrance carrier disposed in the accommodation cavity in a removable manner, where the NFC tag is disposed in the fragrance bottle in an injection molding manner.

In this way, a specific injection molding manner for the NFC tag is provided.

It can be understood that those skilled in the art may determine a specific manner of injection-molding the NFC tag onto the fragrance bottle based on actual conditions. For example, the manner may be as follows: No structural change is made to an existing fragrance bottle, and the NFC tag is directly injection-molded onto an existing structure; or a partial adjustment is made to the structure, so that performance of the fragrance bottle is not affected and complexity of the structure is basically not affected, while it is ensured that the injection molding can be implemented. For example, a part where the injection molding is performed is properly thickened, and so on.

For the foregoing fragrance system based on NFC, in a possible implementation, the fragrance module includes a first printed circuit board (PCB) board, and only the NFC antenna is disposed on the first PCB board.

In this way, adaptability of the fragrance system to use scenarios is improved.

Specifically, the NFC antenna is independently disposed on the first PCB board, and in the case of a subsequent adjustment to an interval between fragrance bottles, it only requires the first PCB board to be adjusted.

For the foregoing fragrance system based on NFC, in a possible implementation, the fragrance module includes a second PCB board disposed separately from the first PCB board, and at least an NFC resonant cavity is disposed on the second PCB board.

For example, the NFC resonant cavity may be independently disposed on the second PCB board, or the NFC resonant cavity and a control module for another function of the fragrance module may be integrated on the second PCB board and share one MCU, thereby achieving lower costs. In the case of a subsequent adjustment to an interval between fragrance bottles, the second PCB board may be further used.

For the foregoing fragrance system based on NFC, in a possible implementation, the housing includes a first cavity and a second cavity, the first PCB board is disposed in the first cavity, and the fragrance chamber is disposed in the second cavity.

In this way, a relationship of relative positions of the first PCB board and the fragrance chamber in the housing is provided.

It can be understood that, to guarantee interference problems such as error communication between adjacent fragrance chambers, circuit design inside the NFC antenna and the NFC tag needs to ensure that an effect area of the NFC antenna is not beyond a corresponding fragrance chamber.

A second aspect of the invention provides a fragrance unit, and the fragrance unit includes: a fragrance bottle formed with an accommodation cavity; and a fragrance carrier disposed in the accommodation cavity in a removable manner, where an NFC tag is disposed on the fragrance bottle in a built-in manner, so that the fragrance bottle and/or the fragrance carrier disposed in the fragrance bottle are/is recognized based on the NFC tag.

In this way, a fragrance unit with an NFC tag is provided, and a specific manner of disposing the NFC tag in the fragrance unit is also provided.

A third aspect of the invention provides a control method of a fragrance system, where the fragrance system includes a fragrance module and a fragrance unit, and the control method includes: recognizing identity information of the fragrance unit by using an NFC tag disposed on the fragrance unit; and enabling the fragrance module when the identity information is recognized, so that a fragrance corresponding to the fragrance unit is sent forth into target space.

In this way, a fragrance unit can be recognized and tracked based on NFC.

A fourth aspect of the invention provides a control method of the fragrance system according to any one of the foregoing technical solutions, and the control method includes: recognizing identity information of the fragrance unit by using an NFC tag disposed on the fragrance unit; and enabling the fragrance module when the identity information is recognized, so that a fragrance corresponding to the fragrance unit is sent forth into target space.

In this way, a fragrance unit can be recognized and tracked based on NFC.

For the foregoing control method of the fragrance system, in a possible implementation, the identity information of the fragrance unit includes scent information and individuality information.

It can be understood that both the scent information and the individuality information are used to represent the identity information of the fragrance unit, and recognition and tracking of the fragrance unit are implemented by combining the two types of information. In addition, both the scent information and the individuality information may include one or more types of information. A specific form of the information may further be a combination of one or more types of forms. Those skilled in the art may also have flexible settings based on actual requirements.

For the foregoing control method of the fragrance system, in a possible implementation, the individuality information is a sequence code corresponding to the scent information.

In this way, a specific form of the individuality information is provided.

It can be understood that the scent information should be understood as information that can be used to distinguish different scents. Therefore, while it is ensured that different scents can be distinguished, a specific form of the information may be determined flexibly based on actual conditions. For example, the scent information may be formed in a manner of using Chinese characters, letters, numbers, symbols, and a combination thereof.

The sequence code should be understood as a code that can be used to at least distinguish a production sequence corresponding to the fragrance unit in the fragrance units of a same scent. Therefore, while it is ensured that the information can be represented, a specific form of the code may be determined flexibly based on actual conditions. For example, similar to the scent information, the sequence code may also be formed in a manner of using Chinese characters, letters, numbers, symbols, and a combination thereof. In addition, on the basis of representing a production sequence, a sequence code may further carry other information such as a production date or a producer. For example, the sequence code is an N^{th} of an M^{th} batch. In this way, if a plurality of fragrance units in a same batch need to be tracked, the tracking can be easily implemented because each fragrance unit in this batch of products corresponds to unique batch information.

It can be understood that the scent information and the sequence code is only a preferred implementation of showing identity information of a fragrance unit, and those skilled in the art may make an adjustment to this manner to some degree based on actual conditions. For example, the adjustment may be: where the sequence code represents only a production sequence of fragrance units, using information about "a production date and a producer" to form a new code that forms the identity information of the fragrance unit together with the foregoing scent information and sequence code; the adjustment may alternatively be: replacing the scent information and/or the sequence code with codes of other forms; and so on.

For the foregoing control method of the fragrance system, in a possible implementation, the scent information is set according to a preset first rule such that a quantity of pieces of scent information is allowed to be enlarged to m; and/or a sequence code corresponding to each piece of scent information is set according to a preset second rule such that a quantity of sequence codes corresponding to each piece of scent information is enlarged to n, and m × n fragrance units are recognizable.

In this way, scents are allowed to be increased in subsequent projects, and distinguishing enough fragrance units is supported, thereby recognizing and tracking more diversified fragrance units without a need to replace a hardware configuration of the fragrance module.

It can be understood that those skilled in the art may set a specific form of the first rule and the second rule flexibly based on actual conditions, so as to reliably increase the scent information and the sequence code and flexibly manage a quantity that can be increased by. Corresponding to the first rule and the second rule, m and n may be determined flexibly subsequently.

For the foregoing control method of the fragrance system, in a possible implementation, the identity information is cryptographic information.

In this way, anti-counterfeiting processing on the NFC tag can implemented. For example, both the scent information and the individuality information are codes. A proper key algorithm is added to the codes and then carried in the NFC tag, so that it is ensured that a whole vehicle recognizes only a fragrance bottle with the built-in NFC tag. In this way, when a counterfeit fragrance bottle is plugged in, the fragrance module may not be enabled.

For the control method of the fragrance system, in a possible implementation, the scent information includes a first-type scent code corresponding to a non-custom scent and/or a second-type scent code corresponding to a custom scent, where when the scent information is the second-type scent code, fragrances of fragrance units with same individuality information are allowed to be changed.

In this way, both the non-custom scent and the custom scent can be recognized.

For the foregoing control method of the fragrance system, in a possible implementation, the scent information includes corresponding name information, and the name information corresponding to the second-type scent code is allowed to be changed.

In this way, while it is ensured that a fragrance unit is recognized, demands of users are better satisfied.

As a preference, because name information of the non-custom scent can basically accurately and properly describe the fragrance unit, the name information of the non-custom scent is not allowed to be changed. However, the custom scent contains an individualized composition, so that initial name information of the custom scent cannot describe the fragrance unit as accurately and properly as the name information of the non-custom scent does. Therefore, the name information of the custom scent is allowed to be changed.

For example, for the custom scent, an initial name may be individualization 1, individualization 2, etc. by default that is not related to an attribute of a fragrance. During use of a user, if there is a requirement of displaying name information of a fragrance unit, the name information may be changed according to the user's demand. For example, "individualization 1" is changed to "favorite".

It should be noted that for each fragrance unit, the change herein is limited to a change unrelated to an end of a link of logical control. For example, the change may be as follows: The displayed name information may be changed only when the name information needs to be displayed in a vehicle or another target scenario. However, even if the displayed name information has been changed, name information corresponding to scent information that is included in whole-vehicle software is not changed.

An example in which a fragrance unit is applied to a vehicle is further used. For example, name information of a fragrance unit is "individualization 1", and after a user changed the name information of the fragrance unit to "favorite", the name information is displayed as "favorite" on a main screen. If the fragrance unit is applied to another vehicle, the name information of the fragrance unit that is read by the vehicle is still "individualization 1", and the foregoing change to "favorite" is not conveyed to the another vehicle simultaneously.

It can be understood that, similar to the foregoing scent information and sequence code, the name information may also be formed in a manner of using Chinese characters, letters, numbers, symbols, and a combination thereof. In addition, the name information may include one or more pieces of information.

A fifth aspect of the invention provides a computer-readable storage medium storing a plurality of program codes, where the program codes are adapted to be loaded by a processor to perform the control method of the fragrance system according to any one of the foregoing aspects.

It can be understood that the computer-readable storage medium has all technical effects of the control method of the fragrance system according to any one of the foregoing aspects. Details are not provided herein again.

A sixth aspect of the invention provides a control apparatus, where the control apparatus includes a processor, and the processor is able to invoke a program and perform the control method of the fragrance system according to any one of the foregoing aspects.

It can be understood that the control apparatus has all technical effects of the control method of the fragrance system according to any one of the foregoing aspects. Details are not provided herein again.

A seventh aspect of the invention provides a vehicle, where the vehicle includes the fragrance system based on NFC according to any one of the foregoing aspects.

It can be understood that the vehicle has all technical effects of the fragrance system based on NFC according to any one of the foregoing aspects. Details are not provided herein again.
Solution 1. A fragrance system based on near field communication (NFC), where the fragrance system includes:
   a fragrance module including a housing and at least one fragrance chamber disposed in the housing, where an NFC antenna is configured above each fragrance chamber; and
   a fragrance unit, which is configured in the fragrance chamber in a removable manner and able to send forth a fragrance corresponding to the fragrance unit into target space when the fragrance module is enabled, where the fragrance unit is provided with an NFC tag that is loaded with identity information of the fragrance unit,
   where the NFC tag is disposed in the fragrance unit in a built-in manner.
Solution 2. The fragrance system based on NFC according to solution 1, where the fragrance unit includes:
   a fragrance bottle formed with an accommodation cavity; and
   a fragrance carrier disposed in the accommodation cavity in a removable manner,
   where the NFC tag is disposed in the fragrance bottle in an injection molding manner.
Solution 3. The fragrance system based on NFC according to solution 1 or 2, where the fragrance module includes a first printed circuit board (PCB) board, and the NFC antenna is disposed on the first PCB board.
Solution 4. The fragrance system based on NFC according to solution 3, where the fragrance module includes a second PCB board disposed separately from the first PCB board, and at least an NFC resonant cavity is disposed on the second PCB board.
Solution 5. The fragrance system based on NFC according to solution 3, where the housing includes a first cavity and a second cavity, the first PCB board is disposed in the first cavity, and the fragrance chamber is disposed in the second cavity.
Solution 6. A fragrance unit, where the fragrance unit includes:
   a fragrance bottle formed with an accommodation cavity; and
   a fragrance carrier disposed in the accommodation cavity in a removable manner,
   where an NFC tag is disposed on the fragrance bottle in a built-in manner, so that the fragrance bottle and/or the fragrance carrier disposed in the fragrance bottle are/is recognized based on the NFC tag.
Solution 7. A control method of a fragrance system, where the fragrance system includes a fragrance module and a fragrance unit, and the control method includes:
   recognizing identity information of the fragrance unit by using an NFC tag disposed on the fragrance unit; and
   enabling the fragrance module when the identity information is recognized, so that a fragrance corresponding to the fragrance unit is sent forth into target space.
Solution 8. A control method of the fragrance system according to any one of solutions 1 to 5, where the fragrance system includes a fragrance module and a fragrance unit, and the control method includes:
   recognizing identity information of the fragrance unit by using an NFC tag disposed on the fragrance unit; and
   enabling the fragrance module when the identity information is recognized, so that a fragrance corresponding to the fragrance unit is sent forth into target space.
Solution 9. The control method of the fragrance system according to solution 8, where the identity information of the fragrance unit includes scent information and individuality information.
Solution 10. The control method of the fragrance system according to solution 9, where the individuality information is a sequence code corresponding to the scent information.
Solution 11. The control method of the fragrance system according to solution 10, where the scent information is set according to a preset first rule such that a quantity of pieces of scent information is enlarged to m; and/or
   a sequence code corresponding to each piece of scent information is set according to a preset second rule such that a quantity of sequence codes corresponding to each piece of scent information is enlarged to n, and m × n fragrance units are recognizable.
Solution 12. The control method of the fragrance system according to solution 10, where the identity information is cryptographic information.
Solution 13. The control method of the fragrance system according to solution 10, where the scent information includes a first-type scent code corresponding to a non-custom scent and/or a second-type scent code corresponding to a custom scent,
   where when the scent information is the second scent code, fragrances of fragrance units with same individuality information are allowed to be changed.
Solution 14. The control method of the fragrance system according to solution 13, where the scent information includes corresponding name information, and the name information corresponding to the second-type scent code is allowed to be changed.
Solution 15. A computer-readable storage medium, storing a plurality of program codes, where the program codes are adapted to be loaded and run by a processor to perform the control method of the fragrance system according to any one of solutions 7 to 14.
Solution 16. A control apparatus, where the control apparatus includes a processor, and the processor is able to invoke a program and perform the control method of the fragrance system according to any one of solutions 7 to 14.
Solution 17. A vehicle, where the vehicle includes the fragrance system based on NFC according to any one of solutions 1 to 5.

### Brief Description of the Drawings

The invention is described below in combination with vehicles and with reference to the accompanying drawings. In the accompanying drawings:
FIG. 1 is a schematic structural diagram 1 of a vehicle fragrance system based on NFC according to an embodiment of the invention;
FIG. 2 is a schematic structural diagram 2 of a vehicle fragrance system based on NFC according to an embodiment of the invention;
FIG. 3 is a schematic structural diagram 3 of a vehicle fragrance system based on NFC according to an embodiment of the invention; and
FIG. 4 is a schematic flowchart of a control method of a vehicle fragrance system according to an embodiment of the invention.

List of reference numerals:
11. First PCB board; 12. Second PCB board; 13. Fragrance chamber; 14. NFC antenna; 15. Cable harness; 2. Fragrance unit; 21. Fragrance bottle; 22. Fragrance stick; and 23. NFC tag.

### Detailed Description of Embodiments

Preferred embodiments of the invention are described below with reference to the accompanying drawings. It should be understood by those skilled in the art that these embodiments are only for explaining the technical principles of the invention and are not intended to limit the scope of protection of the invention, and so on. For example, although the embodiments are described in conjunction with vehicles, obviously, a fragrance unit and a fragrance system and a control method thereof in the invention may be further applied to other scenarios requiring a fragrance to be sent forth.

It should be noted that in the description of the invention, the term "A and/or B" indicates all possible combinations of A and B, for example, only A, only B, or A and B. The term "at least one of A or B" or "at least one of A and B" has a meaning similar to "A and/or B" and may include only A, only B, or A and B. The terms "a/an" and "this" in the singular form may also include the plural form. The terms "first" and "second" are for descriptive purposes only and should not be construed as indicating or implying relative importance.

In addition, for better description of the invention, many details are provided in the following specific implementations, and those skilled in the art should understand that, without some specific details, the invention can still be implemented. In some instances, principles, etc. well known to those skilled in the art are not described in detail in order to highlight the gist of the invention.

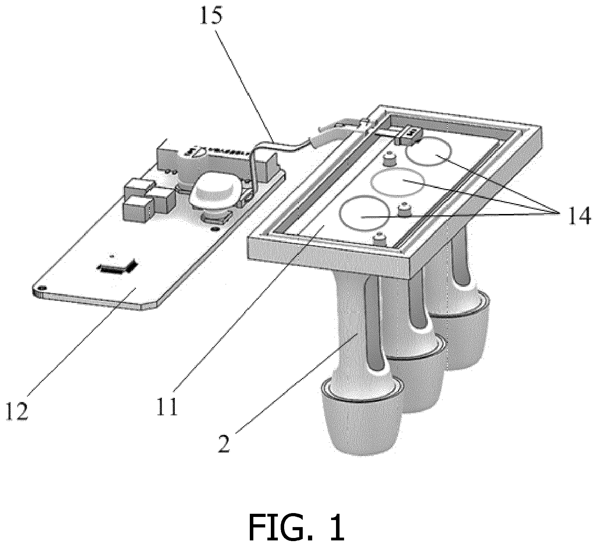

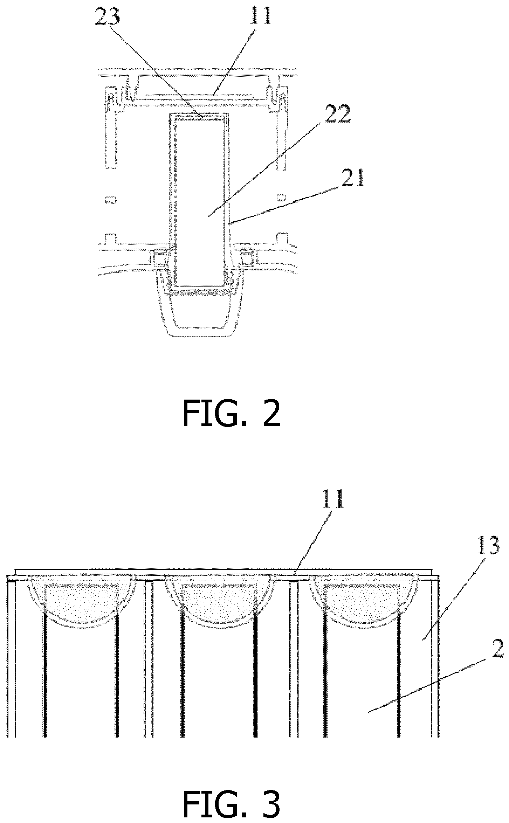

A vehicle fragrance system includes a fragrance module and a fragrance unit that can be disposed in the fragrance module. Referring to FIGs. 1 to 3, FIG. 1 is a schematic structural diagram 1 of a vehicle fragrance system based on NFC according to an embodiment of the invention. FIG. 2 is a schematic structural diagram 2 of a vehicle fragrance system based on NFC according to an embodiment of the invention. FIG. 3 is a schematic structural diagram 3 of a vehicle fragrance system based on NFC according to an embodiment of the invention. As shown in FIGs. 1 to 3, the fragrance system includes a fragrance module and a plurality of fragrance units (including three fragrance units in this embodiment) configured in the fragrance module in a removable manner. The fragrance system in the invention uses NFC to implement recognition of a fragrance unit by the fragrance module before the fragrance module is enabled and when the fragrance module is used.

The fragrance module includes a housing, and the housing includes a first cavity and a second cavity that are vertically disposed. A first printed circuit board (PCB) board 11 is disposed in the first cavity, and three fragrance chambers 13 are disposed in the second cavity. An NFC antenna 14 is configured above each fragrance chamber 13, and the NFC antennas 14 are disposed centrally on the first PCB board 11. A fragrance unit 2 corresponding to a specific type of fragrance (flavor) is disposed in each fragrance chamber 13. The fragrance corresponding to the fragrance unit 2 can be sent forth into a vehicle cabin of the vehicle that is used as target space when the fragrance module is enabled.

The fragrance unit 2 includes a fragrance bottle 21 disposed in the fragrance chamber 13, where the fragrance bottle 21 is formed with an accommodation cavity, and a fragrance stick 22 as a fragrance carrier is disposed in the accommodation cavity in a removable manner. An NFC tag 23 that is loaded with identity information of the fragrance unit 2 is disposed on the fragrance bottle 21 in a built-in manner. For example, the NFC tag 23 is built in on an inner side of the top of the fragrance bottle 21 in an injection molding manner.

As mentioned above, with the NFC antennas 14 being integrated on the first PCB board 11, an NFC resonant cavity and a control module for another function of the fragrance module may be integrated on a second PCB board 12 disposed separately from the first PCB board 11 and share one MCU, thereby reducing costs. The (first and second) PCB boards are connected through a cable harness 15, to implement connection corresponding to an NFC wireless communication technology. In this way, if a parameter such as an interval between fragrance bottles 21 needs to adjusted subsequently, the foregoing second PCB board 12 may be further used. There are two manners of replacing a fragrance: One manner is to replace the fragrance bottle 21 directly, that is, one bottle is for one stick; and the other manner is to replace only the fragrance stick 22 in the fragrance bottle 21, that is, one bottle is for a plurality of sticks.

It can be learned from FIG. 2 that the NFC tag 23 is built in the top of the fragrance bottle 21, and the NFC antenna 14 is located above the fragrance bottle 21. The NFC tag 23 and the NFC antenna 14 are at positions collinear with the axis of the fragrance bottle 21. In this case, circuit design inside the NFC tag 23 and the NFC antenna 14 needs to ensure effectiveness and reliability of the recognition mechanism. Specifically, it needs to be ensured that an effect area of the NFC antenna 14 corresponding to each fragrance bottle 21 is not beyond an effective area of the fragrance chamber 13 in which the NFC antenna 14 is disposed, to avoid problems such as reporting wrong position information of the fragrance bottle 21 caused by error communication with a fragrance bottle 21 in an adjacent fragrance chamber 13.

Identity information (an SN code) is configured for each fragrance bottle 21, where the identity information includes scent information (a CID code) included in the NFC tag 23 and individuality information (a PID code) corresponding to the scent information, and the identity information is mainly for implementing recognition and tracking of the fragrance unit 2. For example, in a possible implementation, the individuality information is a sequence code corresponding to the scent information. The SN code as the identity information includes the CID code as the scent information and the PID code as the sequence code. In other words, the CID code is used to distinguish scents, and the PID code is used to distinguish different fragrance units 2 of a same scent. In this way, only m CID codes need to be reserved, n PID codes are reserved for each CID code, and m × n fragrance units 2 can be distinguished. In addition, each CID code has corresponding name information in whole-vehicle software.

Table 1: Identity information of the fragrance unit and name information corresponding to the identity information

| **Name information** | **CID code** | **PID code** |
|---|---|---|
| Individualization 1 | 0 | From " 1" to "4,294,967,295" |
| Individualization 2 | 1 | From " 1" to "4,294,967,295" |
| Stellar | 2 | From " 1" to "4,294,967,295" |
| Haven | 3 | From " 1" to "4,294,967,295" |
| ··· | ··· | From " 1" to "4,294,967,295" |
| XXX | 253 | From " 1" to "4,294,967,295" |

Referring to Table 1, Table 1 shows specific forms of the identity information of the fragrance unit 2 and the name information corresponding to the identity information according to an embodiment of the invention. As shown in Table 1, a specific form of a first rule is as follows: One byte (8 bits) is reserved for the CID code, such that an extension to a maximum of 254 types of scents can be supported, where only two CID codes are reserved for custom scents, and other CID codes are reserved for non-custom scents. A specific form of a second rule is as follows: Four bytes (32 bits) are reserved for the PID code, such that distinguishing of a maximum of 4,294,967,295 different fragrance bottles 21 of a same scent can be supported.

For example, both the CID code and the PID code are described by using numbers. In this way, a process of determining the identity information is as follows: After a scent is determined, one CID code is given to the scent. For each scent, a plurality of fragrance bottles 21 corresponding to the scent may be produced, and one PID code is given to each fragrance bottle 21. In this way, different fragrance bottle products can be distinguished by using the CID code and the PID code together. Certainly, the CID code and the PID code may be further described in other forms, for example, a combination of numbers and letters, etc.

Still referring to Table 1, one piece of name information may be given to each scent. For example, a correspondence between the CID code and the name information may be written to related whole-vehicle software. Individualization 1 and individualization 2 are name information of custom scents. Stellar, Haven, and other name information not shown are all name information of non-custom scents. In this way, for each fragrance bottle 21, the identity information includes: the CID code as the scent information (for distinguishing scents) and the PID code as the individuality information (for distinguishing different fragrance units of a same scent).

Each piece of scent information has one corresponding piece of name information. For a custom scent, when a corresponding piece of name information needs to be displayed on a main screen of a vehicle, the displayed name information is allowed to be changed. For a non-custom scent, such change is not allowed. For example, name information of a fragrance unit 2 with a CID code being 1 is individualization 2, and name information of a fragrance unit 2 with a CID code being 2 is Stellar. If a user does not perform an extra operation of change, "individualization 2" is displayed on the main screen once the fragrance unit 2 with a CID code being 1 is read, and "Stellar" is displayed on the main screen once the fragrance unit 2 with a CID code being 2 is read. If the user changes "individualization 2" to "favorite", after that, "favorite" is displayed on the main screen once the fragrance unit 2 with a CID code being 1 is read in the vehicle.

Based on a change of the name information, the following operation may be carried out: Once a vehicle owner creates a fragrance stick (which is classified as a custom scent) he/she likes in activities and mount it onto his/her vehicle, since a user is allowed to change name information in his/her own vehicle, participation of the user can be improved. Compared with non-custom scents, prices of custom scents are usually higher, so that such experience can increase a proportion of custom scents that are selected to be mounted, and can further promote development and popularization of a business model corresponding to the custom scents.

A proper key algorithm is added to the scent information and the individuality information in the NFC tag 23, to form the NFC tag 23 that corresponds to the foregoing SN code and that includes a key, such that a whole vehicle can only enable a fragrance module when it is recognized that a fragrance bottle 21 with a tag containing a key is built therein. If a counterfeit fragrance bottle is plugged in, the fragrance module may not be enabled. Because fragrance bottles 21 are after-sale consumable parts, counterfeits are likely to appear in the market. With this setting, use of NFC can implement recognition and tracking of the fragrance bottle 21, and reduce economic losses, caused by counterfeits, of original manufacturers who have developed the fragrance bottle 21. Compromised user experience brought by a user buying counterfeits is also avoided. In addition, a custom scent created by the user can be reliably recognized.

It can be understood that the foregoing CID code and PID code are only used as carriers for conveying information. Therefore, a quantity and a specific meaning of codes are merely specific examples. Those skilled in the art may properly adjust information in the CID code and the PID code based on actual conditions. In addition, information may alternatively be conveyed by using other combination forms of SN codes, to add or change information that is conveyed. For example, the forms may be as follows: In addition to the CID code and the PID code, a code that can convey information about a producer, a production date, etc. of a fragrance module can be added in the SN code; and so on.

The foregoing example in which the SN code includes the CID code and the PID code is still used. In the invention, the fragrance unit 2 of the vehicle includes a first fragrance unit that can send forth a non-custom scent and a second fragrance unit that can send forth a custom scent. Correspondingly, there are two types of scents that can be conveyed by CID codes: A first type is a preset non-custom scent, where for example, the non-custom scent is a classic scent already popular in the market; and a second type is a custom scent, where the custom scent allows a user to participate in its creation.

For example, in a specific implementation, two vehicles provided with the fragrance system described in the invention are randomly selected. In a vehicle 1, the fragrance system includes three fragrance bottles, where one is a first fragrance bottle (denoted as a fragrance bottle a1), and the other two are second fragrance bottles (denoted as fragrance bottles a2 and a3). In a vehicle 2, the fragrance system includes five fragrance bottles, where one is a first fragrance bottle (denoted as a fragrance bottle b1), and the other four are second fragrance bottles (denoted as fragrance bottles b2, b3, b4, and b5).

Because the fragrance bottles (a1 and 21) belong to custom scents, and as described above, only two CID codes are reserved for custom scents (a scent I and a scent II) in a design phase, CID codes of the fragrance bottles (a1 and b1) may be the same or different. It is assumed that the CID codes of the fragrance bottles (a1 and b1) are the same (where for example, both belong to the scent I), PID codes of the fragrance bottles (a1 and b1) are obviously different.

The fragrance bottles (a2, a3, b2, b3, b4, and b5) belong to non-custom scents. It is assumed that the fragrance bottles (a2 and b2) belong to a same scent (where for example, both belong to a scent A), the fragrance bottles (a2 and b2) have the same CID code but different PID codes. It is assumed that the fragrance bottles (a3 and b3) belong to a same scent (where for example, both belong to a scent B), the fragrance bottles (a3 and b3) have the same CID code but different PID codes. It is assumed that the fragrance bottles (b2, b3, b4, and b5) belong to different scents, and therefore, CID codes of the fragrance bottles (b2, b3, b4, and b5) are different. PID codes of the fragrance bottles (b2, b3, b4, and b5) are unrelated, and thus may be the same or different.

As described above, there are two manners of replacing a fragrance in a vehicle cabin:
(1) A first manner is to replace the fragrance bottle 21 directly, that is, one bottle is for one stick. For this manner of replacement, the fragrance unit 2 is changed after the replacement, so that a fragrance after the replacement can be directly determined by using a CID code and a PID code together.
(2) A second manner is to replace only the fragrance stick 22 in the fragrance bottle 21. For this manner of replacement, non-custom scents and custom scents need to be discussed separately. Details are as follows:
(21) For a non-custom scent, before and after the replacement, fragrances of the fragrance stick 22 corresponding to a same CID code are the same, so that a fragrance after the replacement can be directly determined by using the CID code and a PID code together.
(22) For a custom scent, the fragrance stick 22 is custom, so that fragrances of the fragrance stick 22 before and after the replacement are allowed to be different. Because the fragrance bottle 21 is not changed, a CID code and a PID code before and after the replacement remain the same. But because a fragrance of the fragrance stick 22 is allowed to be changed through creation, fragrances before and after the replacement may be different. Correspondingly, the difference may be shown by changing name information again by the user. For example, in the software, name information corresponding to the fragrance bottle 21 with a CID code being 1 is individualization 2, and the user changes a name displayed on a main screen to "favorite" during use of a last fragrance stick 22. Because the fragrance stick 22 is changed, the user may change the name displayed on the main screen to "sweetest".

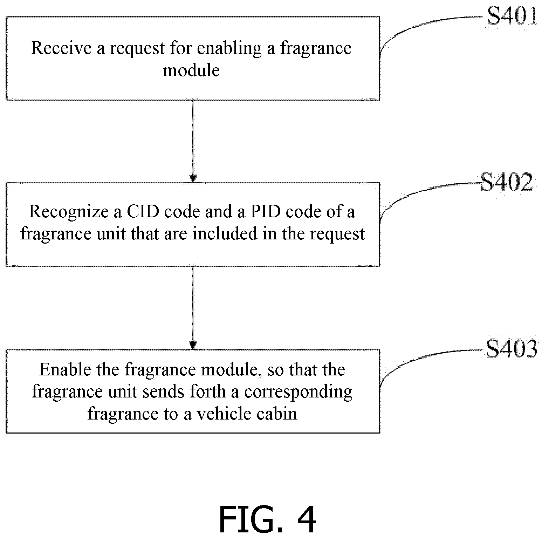

Based on this, referring to FIG. 4, FIG. 4 is a schematic flowchart of a control method of a vehicle fragrance system according to an embodiment of the invention. As shown in FIG. 4, the control method includes:
S401, receiving a request from a user for enabling a fragrance module;
S402, recognizing identity information, included in the request, of a fragrance unit that needs to be enabled,
where for example, the identity information of the fragrance unit includes a CID code and a PID code; and
S403, enabling the fragrance module when the CID code and the PID code are recognized, so that the fragrance unit can send forth a corresponding fragrance to a vehicle cabin.

Each CID code corresponds to one piece of name information. During its use, it is assumed that the fragrance unit is a custom scent, a user can change its name displayed on a main screen of a vehicle based on his/her own demands.

Based on the foregoing control method of the vehicle fragrance system, the vehicle further includes a control module, and may perform the foregoing control method on the vehicle by using the control module.

In the description of the invention, a "module" and a "processor" may include hardware, software, or a combination thereof. A module may include a hardware circuit, various suitable sensors, a communication port, and a memory, or may include a software part, such as program code, or may be a combination of software and hardware. The processor may be a central processing unit, a microprocessor, a graphics processing unit, a digital signal processor, or any other suitable processor. The processor has a data and/or signal processing function. The processor may be implemented in software, hardware, or a combination thereof. A non-transitory computer-readable storage medium includes any suitable medium that can store program code, such as a magnetic disk, a hard disk, an optical disc, a flash memory, a read-only memory, or a random access memory.

Those skilled in the art can understand that in the invention, some or all of the procedures of the control method may alternatively be implemented by a computer program instructing relevant hardware. The computer program may be stored in a computer-readable storage medium. The computer program, when executed by a processor, may implement the steps of the foregoing method embodiments. The computer program includes computer program code, and the computer program code may be in the form of source code, object code, executable file, or some intermediate forms. The computer-readable medium may include: any entity or device, medium, USB flash disk, removable hard disk, magnetic disk, optical disc, computer memory, read-only memory, random access memory, electrical carrier signal, telecommunications signal, software distribution medium, etc. that can carry the computer program code. It should be noted that the content included in the computer-readable medium can be appropriately added or deleted depending on requirements of the legislation and patent practice in a jurisdiction. For example, in some jurisdictions, according to the legislation and patent practice, the computer-readable medium does not include an electrical carrier signal and a telecommunications signal.

Further, it should be understood that, since the configuration of the control module is merely intended to describe functional units of a system in the invention, a physical device corresponding to the control module may be a processor itself, or part of software, part of hardware, or part of a combination of software and hardware in the processor. Therefore, there being one control module is merely exemplary.

It can be understood by those skilled in the art that the control module may be adaptively split based on actual conditions. A specific split form of the control module does not cause the technical solutions to depart from the principle of the invention. Therefore, all technical solutions after the split shall fall within the scope of protection of the invention.

It should be noted that although the steps are described in a specific order in the foregoing embodiment, those skilled in the art can understand that in order to achieve the effects of the invention, different steps are not necessarily performed in this order, but may be performed simultaneously or in another order, or some steps may be added, replaced, or deleted, and these variations all fall within the scope of protection of the invention.

It should be noted that, although an example in which a control method of a fragrance of a vehicle formed in the foregoing specific manner is used for description, those skilled in the art can understand that the invention is not limited to this. In fact, a user can flexibly adjust elements such as related steps and parameters in related steps based on actual application scenarios, etc.

Heretofore, the technical solutions of the invention have been described in conjunction with the preferred embodiments, however, those skilled in the art can readily understand that the scope of protection of the invention is obviously not limited to these specific embodiments. Those skilled in the art could make equivalent changes or substitutions to the related technical features without departing from the principles of the invention, and all the technical solutions after the changes or the substitutions fall within the scope of protection of the invention.

## Claims

1. A fragrance system based on near field communication (NFC), wherein the fragrance system comprises:
a fragrance module comprising a housing and at least one fragrance chamber disposed in the housing, wherein an NFC antenna is configured above each fragrance chamber; and
a fragrance unit, which is configured in the fragrance chamber in a removable manner and able to send forth a fragrance corresponding to the fragrance unit into target space when the fragrance module is enabled, wherein the fragrance unit is provided with an NFC tag that is loaded with identity information of the fragrance unit,
wherein the NFC tag is disposed in the fragrance unit in a built-in manner.

2. The fragrance system based on NFC according to claim 1, wherein the fragrance unit comprises:
a fragrance bottle formed with an accommodation cavity; and
a fragrance carrier disposed in the accommodation cavity in a removable manner,
wherein the NFC tag is disposed in the fragrance bottle in an injection molding manner.

3. The fragrance system based on NFC according to claim 1, wherein the fragrance module comprises a first printed circuit board (PCB) board, and the NFC antenna is disposed on the first PCB board.

4. The fragrance system based on NFC according to claim 3, wherein the fragrance module comprises a second PCB board disposed separately from the first PCB board, and at least an NFC resonant cavity is disposed on the second PCB board.

5. The fragrance system based on NFC according to claim 3, wherein the housing comprises a first cavity and a second cavity, the first PCB board is disposed in the first cavity, and the fragrance chamber is disposed in the second cavity.

6. A fragrance unit, wherein the fragrance unit comprises:
a fragrance bottle formed with an accommodation cavity; and
a fragrance carrier disposed in the accommodation cavity in a removable manner,
wherein an NFC tag is disposed on the fragrance bottle in a built-in manner, so that the fragrance bottle and/or the fragrance carrier disposed in the fragrance bottle are/is recognized based on the NFC tag.

7. A control method of a fragrance system, wherein the fragrance system comprises a fragrance module and a fragrance unit, and the control method comprises:
recognizing identity information of the fragrance unit by using an NFC tag disposed on the fragrance unit; and
enabling the fragrance module when the identity information is recognized, so that a fragrance corresponding to the fragrance unit is sent forth into target space.

8. The control method of the fragrance system according to claim 7, wherein the identity information of the fragrance unit comprises scent information and individuality information, and the individuality information is a sequence code corresponding to the scent information.

9. The control method of the fragrance system according to claim 8, wherein the scent information is set according to a preset first rule such that a quantity of pieces of scent information is enlarged to a number m; and/or
a sequence code corresponding to each piece of scent information is set according to a preset second rule such that a quantity of sequence codes corresponding to each piece of scent information is enlarged to a number n, and m × n fragrance units are recognizable.

10. The control method of the fragrance system according to claim 9, wherein the identity information is cryptographic information.

11. The control method of the fragrance system according to claim 8, wherein the scent information comprises a first-type scent code corresponding to a non-custom scent and/or a second-type scent code corresponding to a custom scent,
wherein when the scent information is the second scent code, fragrances of fragrance units with same individuality information are allowed to be changed.

12. The control method of the fragrance system according to claim 11, wherein the scent information comprises corresponding name information, and the name information corresponding to the second-type scent code is allowed to be changed.

13. A computer-readable storage medium, storing a plurality of program codes, wherein the program codes are adapted to be loaded and run by a processor to perform the control method of the fragrance system according to claim 7.

14. A vehicle, wherein the vehicle comprises the fragrance system based on NFC according to claim 1.
